# EUROPEAN PATENT SPECIFICATION

(11) **EP 1 983 951 B1**
(45) Date of publication and mention of the grant of the patent: **20.02.2019**
(21) Application number: 06850980.1
(22) Date of filing: 18.12.2006
(51) Int. Cl.: A61F 13/00, A61M 27/00, A61L 15/18, A61L 15/42

(54) **ADSORBENT-CONTAINING HEMOSTATIC DEVICES**
HÄMOSTATISCHE VORRICHTUNGEN MIT ADSORPTIONSMITTELN
DISPOSITIFS HÉMOSTATIQUES CONTENANT UN ADSORBANT

(30) Priority: 30.12.2005 US 755044 P; 13.12.2006 US 609952; 13.12.2006 US 610443
(43) Date of publication of application: 29.10.2008
(73) Proprietor: UOP LLC, P.O. BOX 5017, Des Plaines IL 60017-5017 (US)
(72) Inventor: WILCHER, Steve A., Des Plaines, Illinois 60017-5017 (US); BEDARD, Robert L., Des Plaines, Illinois 60017-5017 (US)
(74) Representative: Crooks, Elizabeth Caroline
(86) International application number: PCT/US2006/062214
(87) International publication number: WO 2007/120342

(56) References cited:
- EP-A1- 1 738 779
- EP-A1- 1 797 850
- EP-A2- 1 810 697
- WO-A1-01/64148
- WO-A1-02/30479
- WO-A2-2006/088912
- WO-A2-2007/081996
- US-A- 4 826 497
- US-A- 5 650 221
- US-A- 5 856 245
- US-A- 5 875 164
- US-A- 6 102 107
- US-A1- 2005 074 505
- DATABASE WPI Week 200220 Thomson Scientific, London, GB; AN 2002-151557 XP002674712, & JP 2001 322926 A (SEKISUI CHEM IND CO LTD) 20 November 2001 (2001-11-20)
- DATABASE WPI Week 198546 Thomson Scientific, London, GB; AN 1985-286304 XP002674713, & JP 60 195452 A (NIPPON PAINT CO LTD) 3 October 1985 (1985-10-03)

## Description

### BACKGROUND OF THE INVENTION

This invention relates generally to the use of inorganic materials and adsorbents to stop bleeding. More particularly, this invention relates to inorganic materials and adsorbents incorporated into adsorbent media such as nonwoven materials or films where a sufficient amount of these adsorbents are present to stop blood loss while minimizing increased temperature resulting from the adsorption of water by the adsorbents.

Wounds are generally classified as acute or chronic in accordance with their healing tendencies. Acute wounds from trauma or surgery include wounds such as active bleeding wound sites, e.g., wounds that have detectable, unclotted blood. The rapid control of topical bleeding at active bleeding wound sites is of critical importance in wound management, especially for the management of trauma, e.g., as a result of military exercises or surgery.

Conventional approaches such as manual pressure, cauterization, or sutures may be time consuming and are not always effective in controlling bleeding. Trauma care has received great attention recently as United States troops on a daily basis face combat situations that result in wounds accompanied by significant blood loss. In many cases, the individual may have been able to survive the initial injury only to die of blood loss. Given the central role of hemostasis in trauma care, a great deal of attention has been focused on developing products that can rapidly induce clotting, stop the bleeding, form a tight bond to the wound surface, facilitate scab formation and be compatible with the host tissue. Currently there are several categories of products being used that can be differentiated by their mechanism of action. The first category includes materials that accelerate the coagulation process by absorbing water from the blood. The products in this category include basic cotton gauze. Also within this category are products from Johnson & Johnson's Ethicon division that sells its Surgicel™ regenerated cellulose product line in various forms. There are other cellulosic type products in the marketplace. The second category of products seeks to enhance coagulation by adding features that can increase clotting enzymatic activity. Such products may include such components as thrombin, fibrinogen, propyl gallate, aluminum sulfate, fully acetylated glucosamine and ε-aminocaproic acid. Other hemostatic agents have difficulty adhering to wet tissue and lack a framework onto which a clot can adhere. Z-Medica's zeolite based QuikClot™ product has proven effective in stopping bleeding but may cause the patient to be exposed to excess heat and the use of the powder form of this product necessitates that the product be washed from the wound after clotting is complete.

Each of these prior art products are deficient in at least one aspect. Products that function solely through absorption of water from the blood tend not to be particularly selective in concentrating the blood constituents useful in clotting such as platelets, erythrocytes and plasmas and therefore are not as effective as other products in enhancing coagulation. The second category of products enhance coagulation by adding components such as thrombin, fibrinogen, propyl gallate, aluminum sulfate, fully acetylated glucosamine and ε-aminocaproic acid that increase clotting enzymatic activity. While these products can be very effective at stopping bleeding they can also be quite expensive, have shelf life limitations and in some cases where the components are derived from animals or humans may offer a mechanism for pathogen transfer or allergic reaction. In the third product category, the HemCon product suffers from potential allergenic side effects, short shelf life and high cost. The Z-Medica QuikClot product suffers from problems with high heat of adsorption that can cause significant discomfort to users and limits its utility in heat sensitive parts of the body. The product, while quite selective in adsorbing water from the blood, is not the optimal product to concentrate those blood constituents which enhance coagulation since the product is literally poured onto the wound and must then be carefully washed from the injury.

A hemostatic material that is biocompatible, provides superior hemostasis, and that can be fabricated into a variety of forms suitable for use in controlling bleeding from a variety of wounds is still sought. This type of hemostatic material is sought for both surgical applications as well as in field treatment of traumatic injuries. In vascular surgery, due to the involvement of the blood vessels, bleeding is particularly problematic. In cardiac surgery, the multiple vascular anastomoses and cannulation sites, complicated by coagulopathy induced by extracorporeal bypass, can result in bleeding that can only be controlled by topical hemostats. Rapid and effective hemostasis during spinal surgery, where control of osseous, epidural, and/or subdural bleeding or bleeding from the spinal cord is not amenable to sutures or cautery, can minimize the potential for injury to nerve roots and reduce the procedure time. In liver surgery, for example, live donor liver transplant procedures or removal of cancerous tumors, there is a substantial risk of continued bleeding. An effective hemostatic material can significantly enhance patient outcome in such procedures. Even in those situations where bleeding is not massive, an effective hemostatic material can be desirable, for example, in dental procedures such as tooth extractions and other oral surgery, as well as the treatment of abrasions, burns, and the like.

There remains a need for an effective hemostatic product that can be delivered in an easy to use form. Prior to the present invention, porous carriers or porous articles, e.g. non-woven fibrous articles containing molecular sieves and hydrophilic oxides

In the treatment of certain conditions and during some surgeries, in order to prevent coagulation of a patient's blood, anticoagulants are routinely administered; the most common of which is heparin. Heparin can be administered in high concentrations during periods of extracorporeal circulation during surgeries such as open heart surgery. During these procedures, the Activated Clotting Time (ACT) and other endpoint based coagulation assays are frequently used to monitor these high levels of heparin and other coagulation parameters.

In 1966, Dr. Paul Hattersley, a physician from California, outlined the design and usage of a fresh whole blood clotting test utilizing a particulate for contact activation. This was to facilitate rapid test conclusion in a clinically meaningful timeframe. The test Hattersley described included placing 1 ml or more of blood into a tube prefilled with 12 mg of activator (diatomaceous earth, Celite®). This tube was prewarmed to body temperature (37°C) prior to administration of the patient blood sample. A timer was started when blood first entered the test tube. The tube was filled, and inverted a few times to accommodate mixing. The tube was then placed into a 37°C water bath. At one minute and at every 5 seconds thereafter the tube was removed from the water bath and tilted so that the blood spread the entire length of the tube. The timer was stopped at the first unmistakable signs of a clot. Modifications have been made to the ACT test that determines clotting ability of whole blood over the years including improved instrumentation. A variety of activators are used in the test, including diatomaceous earth, kaolin, glass beads and colloidal silica. A similar test known as the APTT (activated partial thromboplastin time procedure) is used to test the clotting capacity of blood plasma. While the ACT test was first developed over 40 years ago, it only has recently been found by the same research group as have the present invention and described in a patent application filed the same day as this application, that the types of
activators that are used to test the coagulation of blood in the laboratory are exceedingly effective in clotting blood from wounds in humans and animals.

WO 02/30479 and WO 01/64148 disclose hemostatic articles comprising inorganic materials.

There remains a need for an effective hemostatic product that can be delivered in an easy to use form. Prior to the present invention, porous carriers or porous articles, e.g. non-woven fibrous articles containing inorganic materials have not been used as hemostatic devices. Such hemostatic articles comprising molecular sieves or inorganic materials have now been found to provide ease of application, effective hemostasis, and reduction in exposure of the patient to high temperature increases owing to high heats of adsorption. These products are also useful in surgical applications that were not available using a powdered molecular sieve or hydrophilic oxide product.

### SUMMARY OF THE INVENTION

The scope of the invention is defined by the claims. Any references in the description to methods of treatment refer to the compounds, pharmaceutical compositions and medicaments of the present invention for use in a method for treatment of the human (or animal) body by therapy.

By using a hemostatic article comprising an adsorbent or inorganic material that provides hemostatic properties and a porous carrier, where the carrier is a woven or non-woven fibrous article that is made from aramid fibers, the invention addresses virtually all of the shortfalls of the products described above. Incorporation of the active ingredient into a porous carrier addresses the problems experienced with zeolite containing products such as Z-Medica's QuikClot. The heat of adsorption can be quickly conveyed away from the point of use of the hemostatic article and as a result the user should experience virtually no discomfort as a result of the temperature rise owing to the heat of adsorption. The active ingredient is fully contained in the porous carrier allowing for minimal clean-up to remove the product from the wound.

When the porous carrier is a sheet (and adsorbent) which includes a fibrillated, high surface area fiber and a material that is highly selective for water can result in more effective concentration of blood constituents that further enhance coagulation beyond that observed for other products. The adsorbent containing porous sheet that conforms to irregular surfaces can also he readily used in difficult to access wounds and injuries. Other features desirable in a wound dressing such as biocidal activity can be incorporated either directly into the sheet or into a dressing that includes such a sheet.

### DETAILED DESCRIPTION OF THE INVENTION

Hemostasis is the arresting of bleeding, whether by normal vasoconstriction, by an abnormal obstruction, by coagulation or surgical means. Hemostasis by coagulation (which is the subject of the products of the present invention) is dependent upon a complex interaction of plasma coagulation and fibrinolytic proteins, platelets, and the blood vasculature. The present invention provides compositions and materials that react with the hemostatic system to treat or prevent bleeding. In particular, the compositions and materials of preferred embodiments result in coagulation of blood.

Effective delivery of hemostatic agents to wounds is particularly desirable in the treatment of injuries characterized by arterial or venous bleeding, as well as in surgical procedures where the control of bleeding can become problematic, e.g., large surface areas, heavy arterial or venous bleeding, oozing wounds, and in organ laceration or resectioning. The compositions and materials of preferred embodiments can possess a number of advantages in delivery of hemostatic agents to wounds, including but not limited to, ease of application and removal, bioadsorption potential, suturability, antigenicity, and tissue reactivity.

Depending upon the nature of the wound and the treatment method employed, the devices of the present invention can employ different forms. For example, a puff, e.g. cotton ball, fleece, or sponge form can be preferable for controlling the active bleeding from artery or vein, or for internal bleeding during laparoscopic procedures. In neurosurgery, where oozing brain wounds are commonly encountered, a sheet form of the hemostatic article can be preferred. Likewise, in oncological surgery, especially of the liver, it can be preferred to employ a sheet form or sponge form of the hemostatic article, which is placed in or on the tumor bed to control oozing. In dermatological applications, a sheet form can be preferred. In closing punctures in a blood vessel, a puff form is generally preferred. A suture form, such as a microsuture or a macrosuture, can be preferred in certain applications. Despite differences in delivery and handling characteristic of the different forms, the devices are effective in deploying hemostatic agents to an affected site and to rapidly initiate hemostatic plug formation through platelet adhesion, platelet activation, and blood coagulation.

The materials which can be used as the porous carriers for the adsorbent or inorganic hemostatic material are articles which can support an effective amount of adsorbent and can be applied to the particular wound being treated. The porous carrier is a woven or non-woven fibrous article that is made from aramid fibers. Adsorbent or inorganic material containing non-woven articles can be prepared by textile-, paper-, extrusion type and a combination or hybrid of processes. The product is prepared using aramid fibers. Various binders can also be used in preparing the sheets, some of which may have functional groups that can aid in the release of coagulation enhancing agents.

The adsorbents or inorganic materials which can be used to form the hemostatic article are any of those which are effective in blood clotting. Non-limiting examples of these adsorbents are zeolitic molecular sieves and non-zeolitic molecular sieves. The inorganic materials can include diatomaceous earth, glass powder or fibers, precipitated or fumed silica, montmorillonite clays, kaolin. Zeolites are crystalline aluminosilicate compositions which are microporous and which are have a three-dimensional oxide framework formed from corner sharing AlO₂ and SiO₂ tetrahedra. Both naturally occurring and synthetic zeolites can be used. Non limiting examples of zeolites which can be used are the family of zeolites of structure type X, Y, A, beta, etc. Included in these zeolites are the as synthesized zeolites and those that have been Ca exchanged with other cations, e.g. Ca. Non-zeolite molecular sieves are those which do not contain both Al₂O₃ and SiO₂ tetrahedra as essential framework constituents, but which exhibit the ion-exchange and/or adsorption characteristics of the zeolites.

In some embodiments of the present invention, the selected adsorbents have lower heat of adsorption than other adsorbents that may be effective but result in higher heat of adsorption in use. The adsorbents or inorganic materials can be loaded into the sheets across a wide range of concentrations spanning 1 wt-% to over 95 wt-%. The size of the fibers and adsorbents or inorganic materials used to prepare the sheets can be varied across a wide range starting as low as nanoscale materials to formed beads or crushed extrudate. To achieve an active and functional product the sheet may need to be heat activated to drive off residual moisture. The product is then enclosed in a sealed container until needed for use. The adsorbent or inorganic material containing sheet can then be applied directly to the point of injury where its benefits in accelerating coagulation are achieved.

Preferably, the fibers employed in the present invention are fibrillated to increase the surface area and the capacity to retain higher loadings of adsorbents or inorganic materials and other additives, when determined to be desirable to obtain the desired functionality.

A single hemostatic substrate or combination of hemostatic substrate comprising the porous carrier of the present invention can be employed. Different substrate forms can be preferred, for example, puff, fleece, fabric or sheet, sponge, suture, or powder. In this specification, the term "fleece" is used as a broad term in accordance with its ordinary meaning and includes any fibrous material treated to be flexible, malleable or the like. A fleece may be provided, without limitation, in a non-woven form or in a puff, ball or sheet form. It is to be understood that the fibrous fleece can be treated or coated in any suitable manner to enhance its hemostatic properties. The term "puff' is also used as a broad term in accordance with its ordinary meaning and includes any fibrous material arranged into a soft ball or pad. A puff may be constructed using a fleece. The term "sponge" is also used as a broad term in accordance with its ordinary meaning and includes a material configured to absorb fluids such as blood. A sponge may be constructed using, without limitation, a fleece, puff, fiber, fabric or the like alone or in combination with another material. A homogeneous mixture of different substrate-forming materials can be employed, or composite substrates can be prepared from two or more different formed substrates. In certain embodiments, it can be desirable to add an auxiliary hemostatic agent to the adsorbent or inorganic material hemostatic agents used in the present invention. Any suitable hemostatic agent can be deposited upon the substrates of preferred embodiments. Among the auxiliary hemostatic agents that can be used are bioabsorbable microporous polysaccharide microspheres, clotting factor concentrates, recombinant Factor VIIa (NOVOSEVEN®); alphanate FVIII concentrate; bioclate FVIII concentrate; monoclate-P FVIII concentrate; haemate P FVIII; von Willebrand factor concentrate; helixate FVIII concentrate; hemophil-M FVIII concentrate; humate-P FVIII concentrate; hyate-C®. Porcine FVIII concentrate; koate HP FVIII concentrate; kogenate FVIII concentrate; recombinate FVIII concentrate; mononine FIX concentrate; and fibrogammin P FXIII concentrate. Such hemostatic agents can be applied to the substrate in any suitable form (powder, liquid, in pure form, in a suitable excipient, on a suitable support, or the like).

A single hemostatic agent or combination of hemostatic agents can be employed. Preferred loading levels for the hemostatic agent on the substrate can vary, depending upon the nature of the substrate and hemostatic agent, the form of the substrate, and the nature of the wound to be treated.

Hemostatic fabrics can be prepared from fibers according to the method described above for preparation of hemostatic puffs. It is generally preferred that one side of the fabric has a smooth surface and the other side of the fabric has a rough surface. However, in certain embodiments, a fabric having two rough sides can be preferred, such as, for example, for use in connection with an irregular wound, or a deep wound, such as a potentially lethal groin injury. In preferred embodiments, the rough surface is exposed to the wound so as to maximize contact of the fibers with the wound, resulting in an improved hemostatic effect and superior adherence to the wound as well as contact of the adsorbents with the blood flowing from the wound. In preparing a hemostatic fabric comprising fibers loaded with adsorbents or inorganic materials, it is generally preferred that the resulting fabric contain from 1 to 95 wt-% adsorbents or inorganic materials, more preferably from 5 wt-% to 90 wt-% adsorbents or inorganic materials and most preferably from 50 wt-% to 80 wt-%. In certain embodiments, however, higher or lower levels of adsorbents can be preferred. If an additional hemostatic agent is employed, or other components are to be added to the fibers or other substrate, different loading levels can be preferred.

The hemostatic fabric can be provided in the form of a sheet of a pre-selected size. Alternatively, a larger sheet of hemostatic fabric can be cut, trimmed, or folded to provide a size and shape appropriate to the wound. Alternatively, trimmed sheets can be stacked into multilayers or laminates. Although the hemostatic fabric is biocompatible in cutaneous or topical applications, it can be removed from the wound after a satisfactory degree of hemostasis is achieved, or it can be left in place until the wound is healed. Hemostatic fabric can be useful as artificial skin, and/or can provide antibiotic properties. A hemostatic sponge can be prepared according to methods known in the art for preparing a porous sponge from a biocompatible or bioabsorbable polymeric material. Such methods typically involve preparation of a solution of the polymeric material, crosslinking agents, and foaming agents. The sponge can be loaded with an adsorbent hemostatic agent during formation of the sponge.

While it is generally preferred to apply the hemostatic material (for example, a hemostatic fabric, sponge, puff, or powder prepared as described above) directly to the wound, and while the hemostatic material exhibits satisfactory adhesion to many types of wounds, in certain embodiments it can be preferred to incorporate the hemostatic material into a wound dressing including other components.

To ensure that the hemostatic material remains affixed to the wound, a suitable adhesive can be employed, for example, along the edges of one side of the hemostatic fabric, sponge or puff. Although any adhesive suitable for forming a bond with skin can be used, it is generally preferred to use a pressure sensitive adhesive. Pressure sensitive adhesives are generally defined as adhesives that adhere to a substrate when a light pressure is applied but leave no residue when removed. Pressure sensitive adhesives include, but are not limited to, solvent in solution adhesives, hot melt adhesives, aqueous emulsion adhesives, calenderable adhesive, and radiation curable adhesives. Solution adhesives are preferred for most uses because of their ease of application and versatility. Hot melt adhesives are typically based on resin-tackified block copolymers. Aqueous emulsion adhesives include those prepared using acrylic copolymers, butadiene styrene copolymers, and natural rubber latex. Radiation curable adhesives typically consist of acrylic oligomers and monomers, which cure to form a pressure sensitive adhesive upon exposure to ultraviolet lights.

The most commonly used elastomers in pressure sensitive adhesives include natural rubbers, styrene-butadiene latexes, polyisobutylene, butyl rubbers, acrylics, and silicones. In preferred embodiments, acrylic polymer or silicone based pressure sensitive adhesives are used. Acrylic polymers generally have a low level of allergenicity, are cleanly removable from skin, possess a low odor, and exhibit low rates of mechanical and chemical irritation. Medical grade silicone pressure sensitive adhesives are preferred for their biocompatibility.

Amongst the factors that influence the suitability for a pressure sensitive adhesive for use in wound dressings of preferred embodiments is the absence of skin irritating components, sufficient cohesive strength such that the adhesive can be cleanly removed from the skin, ability to accommodate skin movement without excessive mechanical skin irritation, and good resistance to body fluids. In preferred embodiments, the pressure sensitive adhesive comprises a butyl acrylate. While butyl acrylate pressure sensitive adhesives are generally preferred for many applications, any pressure sensitive adhesive suitable for bonding skin can be used. Such pressure sensitive adhesives are well known in the art.

As discussed above, the hemostatic materials of preferred embodiments generally exhibit good adherence to wounds such that an adhesive, for example, a pressure sensitive adhesive, is not necessary. However, for ease of use and to ensure that the hemostatic material remains in a fixed position after application to the wound, it can be preferable to employ a pressure sensitive adhesive.

While the hemostatic fabrics and other hemostatic materials of preferred embodiments generally exhibit good mechanical strength and wound protection, in certain embodiments it can be preferred to employ a backing or other material on one side of the hemostatic material. For example, a composite including two or more layers can be prepared, wherein one of the layers is the hemostatic material and another layer is, e.g., an elastomeric layer, gauze, vapor-permeable film, waterproof film, a woven or nonwoven fabric, a mesh, or the like. The layers can then be bonded using any suitable method, e.g., adhesives such as pressure sensitive adhesives, hot melt adhesives, curable adhesives, and application of heat or pressure such as in lamination, physical attachment through the use of stitching, studs, other fasteners, or the like.

Advantage can be taken of the surface charge characteristics of the fibers and fillers by ion exchanging additional functional ions such as Ca++ to aid coagulation. Addition of anionic polyelectrolytes may also add ion exchange capacity. The fiber composition and its degree of fibrillation can also be varied to enhance and optimize coagulation. Additional bioactive fillers such as active glasses that release Ca, Ag ions can also be incorporated into the sheets.

One of the more effective embodiments of the present invention involves incorporating 5A powder and micro-fibrillated aramid fiber into a non-woven sheet prepared using the paper making process technique. The zeolite loading of the sheet is in the range of 65 to 75 wt-%. The sheet is activated at temperature under a nitrogen atmosphere and then stored in a sealed air tight container. The sheet is then removed from the container and applied directly to the wound to stop the bleeding. Once the bleeding has been stopped and the patient stabilized the wound can be further cleaned.

It is believed that the hemostatic devices of the invention do not require an additional hemostatic agent to function effectively to control bleeding, including hemorrhage of an important internal organ. As a result, the hemostatic devices of the invention which do not further contain a hemostatic agent have good thermal stability and can be stored for months to a few years without refrigeration and loss of effectiveness. Such embodiments of the invention are useful for various medical situations and are particularly useful for field and emergency use, since each may be stored in a ready-to-use state for a lengthy period, even in the absence of refrigeration. Such devices of the invention also are less expensive to make and/or use compared to hemostatic devices which contain a further hemostatic agent to achieve a comparable level of hemostatic activity. In certain embodiments, the hemostatic devices of the invention further include a therapeutically effective amount of one or more therapeutic agents, such as an agent which promotes wound-healing. Agents which promote wound-healing include anti-inflammatory agents such as agents which inhibit leukocyte migration into the area of surgical injury, anti-histamines; agents which inhibit free radical formation; and bacteriostatic or bacteriocidal agents. In general, a therapeutically effective amount means that amount necessary to delay the onset of, inhibit the progression of, or halt altogether the particular condition being treated. Generally, a therapeutically effective amount will vary with the subject's age, condition, and sex, as well as the nature and extent of the condition in the subject, all of which can be determined by one of ordinary skill in the art. The dosage of therapeutic agent contained in the hemostatic devices of the invention may be adjusted to accommodate the particular subject and condition being treated. As used herein,
the phrase, "agents which promote wound-healing" refers to agents, the administration of which, promote the natural healing process of a wound. Agents that promote wound-healing include anti-inflammatory agents, agents which inhibit free radical formation, and bacteriostatic or bacteriocidal agents.

Anti-inflammatory agents are agents which inhibit or prevent an immune response in vivo and include: (i) agents which inhibit leukocyte migration into the area of surgical injury ("leukocyte migration preventing agents"), and anti-histamines. Representative leukocyte migration preventing agents include silver sulfadiazine, acetylsalicylic acid, indomethacin, and Nafazatrom. Representative anti-histamines include pyrilamine, chlorpheniramine, tetrahydrozoline, antazoline, and other anti-inflammatories such as cortisone, hydrocortisone, beta-methasone, dexamethasone, fluocortolone, prednisolone, triamcinolone, indomethacin, sulindac, its salts and its corresponding sulfide, and the like.

Representative agents which inhibit free radical formation include antioxidants that inhibit the formation and/or action of oxide products, superoxide dismutase (SOD), catalase, glutathione peroxidase, b-carotene, ascorbic acid, transferrin, ferritin, ceruloplasmin, and desferrioxamine α-tocophenol.

Representative bacteriostatic or bacteriocidal agents include antibacterial substances such as β-lactam antibiotics, such as cefoxitin, n-formamidoyl thienamycin and other thienamycin derivatives, tetracyclines, chloramphenicol, neomycin, gramicidin, bacitracin, sulfonamides; aminoglycoside antibiotics such as gentamycin, kanamycin, amikacin, sisomicin and tobramycin; nalidixic acids and analogs such as norfloxican and the antimicrobial combination of fluoroalanine/pentizidone; nitrofurazones, and the like.

The hemostatic devices of the invention can contain one or more therapeutic agents, alone or in combination with one or more hemostatic agents.

Various additives, optionally, can be incorporated into the hemostatic devices of the invention without substantially reducing the hemostatic activity of these devices. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled within the woven sheets of the present invention, and with each other, in a manner such that there is no interaction which would substantially impair the desired hemostatic activity.

Additives (e.g., retention aids) and binders known in the art of specialty paper making were added together or sequentially to the molecular sieve slurry to improve the retention of the molecular sieve components within the fiber matrix, and to improve paper strength. Such additives include starch, poly-vinyl alcohols (PVA), acrylics, and microcrystalline cellulose (e.g., carboxymethyl cellulose). The organic additives are added in an amount of 5% by weight of fiber plus molecular sieve on a 200°C basis. These additives may be added to the makeup tank with the refined fiber and/or added to the head box. The preferred flocculation system is a dual chemical recipe which includes poly-DADMAC coagulant, a cationic, low molecular weight polyelectrolyte, and anionic polyacryamide (a-PAM), a high molecular weight polyelectrolyte.

Paper can be prepared from aramid pulps made by both DuPont and Twaron. Paper samples may contain inorganic material or molecular sieve filler content from 75 to 90 wt-%.

### EXAMPLE

The effectiveness of the hemostatic articles of the present invention was tested as shown in the following example of calcium exchanged zeolite. Sheets of paper were prepared with a mixture of 4.84 grams aramid pulp blended with liters of water which was mixed together very thoroughly. Then 5.09 grams of calcium ZB-100 zeolite (UOP LLC, Des Plaines, IL) was added and thoroughly mixed. 0.4526 Grams of DADMAC (diallyldimethylammonium chloride) was added with further mixing. The mixture was diluted to 3.0 liters with water and added to a Techpap paper making machine. Also added was Percol E38 solution which was diluted 100 times with 0.325 wt-% solids. A sheet of paper was cast and rolled and dried. The paper was then tested for its hemostatic capabilities according to the following procedure.

The following protocol was used to test the blood samples.

The apparatus that was used was a TEG® analyzer from Haemoscope Corp. of Morton Grove, Illinois. This apparatus measures the time until initial fibrin formation, the kinetics of the initial fibrin clot to reach maximum strength and the ultimate strength and stability of the fibrin clot and therefore its ability to do the work of hemostasis - to mechanically impede hemorrhage without permitting inappropriate thrombosis.
On unactivated samples:
i. Pipet 360 uL from red topped tube into cup, start TEG test
On activated samples:
i. First, obtain the zeolite-containing paper sample to be tested from lab. They should be weighed, bottled, oven activated (if needed), and capped prior to the start of the experiment. Zeolite-containing paper samples are bottled in twice the amount that need to be tested. For example, if channel 2 is to test 5 mg of zeolite-containing paper and blood, the amount weighed out in the bottle for channel two will be 10 mg. For 10 mg samples, 20 mg is weighed out, etc. See note below for reason.
ii. For one activated run, three zeolite-containing paper samples were tested at a time. An unactivated blood sample with no additive is run in the first channel. Channels 2, 3 and 4 are blood samples contacted with zeolite-containing paper.
iii. Once ready to test, set one pipet to 720 uL and other pipet to 360 uL. Prepare three red capped tubes (plain polypropylene-lined tubes without added chemicals) to draw blood and prepare three red additional capped tubes to pour zeolite-containing paper sample into.
iv. Draw blood from volunteer and bring back to TEG analyzer. Discard the first tube collected to minimize tissue factor contamination of blood samples. Blood samples were contacted with zeolite-containing paper material and running in TEG machine prior to an elapsed time of 4-5 minutes from donor collection.
v. Open bottle 1 and pour zeolite-containing paper into red capped tube.
vi. Immediately add 720 uL of blood to zeolite-containing paper in tube.
vii. Invert 5 times.
viii. Pipet 360 uL of blood and zeolite-containing paper mixture into cup.
ix. Start TEG test.

Note: The proportions are doubled for the initial mixing of blood and zeolite-containing paper because some volume of blood is lost to the sides of the vials, and some samples absorb blood. Using double the volume ensures that there is at least 360 uL of blood to pipet into cup. The proportion of zeolite-containing paper to blood that we are looking at is usually 5mg/360uL, 10mg/360uL, and 30mg/360uL

The R(min) reported in the Table below is the time from the start of the experiment to the initial formation of the blood clot as reported by the TEG analyzer. The TEG® analyzer has a sample cup that oscillates back and forth constantly at a set speed through an arc of 4°45'. Each rotation lasts ten seconds. A whole blood sample of 360 ul is placed into the cup, and a stationary pin attached to a torsion wire is immersed into the blood. When the first fibrin forms, it begins to bind the cup and pin, causing the pin to oscillate in phase with the clot. The acceleration of the movement of the pin is a function of the kinetics of clot development. The torque of the rotating cup is transmitted to the immersed pin only after fibrin-platelet bonding has linked the cup and pin together. The strength of these fibrin-platelet bonds affects the magnitude of the pin motion, such that strong clots move the pin directly in phase with the cup motion. Thus, the magnitude of the output is directly related to the strength of the formed clot. As the clot retracts or lyses, these bonds are broken and the transfer of cup motion is diminished. The rotation movement of the pin is converted by a mechanical-electrical transducer to an electrical signal which can be monitored by a computer.

The resulting hemostasis profile is a measure of the time it takes for the first fibrin strand to be formed, the kinetics of clot formation, the strength of the clot (in shear elasticity units of dyn/cm²) and dissolution of clot.

| Sample | R(min) |
|---|---|
| Run 1 Native - no paper added | 25.2 |
| Run 1-vial act Ca A paper 10 mg | 6.7 |
| Run 1-vial act Ca A paper 30 mg | 4.2 |
| Run 1-vial act Ca A paper 5 mg | 10.2 |
| Run 2 Native - no paper added | 34.5 |
| Run 2 - vial act CaA paper 30 mg | 3.6 |
| Run 2 - vial act CaA paper 5 mg | 6.5 |
| Run 2 - vial act CaA paper 10 mg | 5.5 |

The inorganic hemostatic materials that can be used include the following: A mesoporous bioactive glass with a calcium silicate composition that is prepared as follows:
Mixture A - 15 g. of tetraethylorthosilicate, 5.0 g. calcium nitrate tetrahydrate, 20.1 g. of ethanol, 7.5 g deionized water, and 2.5 g. 1 M HCl.
Mixture B - A triblock copolymer solution was made by dissolving 20.02 g of Pluronic P123 triblock copolymer (BASF) in 80.12 g of ethanol.
Mixture C - 45 ml of Mixture B would be added to Mixture A and stirred by magnetic stirring for two minutes. The mixture would then heated in an open porcelain crucible at 60°C for 16 hours, then placed in a furnace and heated at 3°C per minute to 550°C, held at 550°C for four hours, then cooled to 100°C. The material would then removed from the furnace and cooled to room temperature.

Diafil 460 is a high surface area ∼30m²/g diatomaceous earth that is available from World Minerals Inc. is headquartered in Santa Barbara, California, USA

A Ca-silicate sol-gel glass can be synthesized by adding 46.8 ml of tetraethylorthosilicate, 21.43 g of calcium nitrate tetrahydrate, 45 ml of deionized water, and 7.6 ml of 2 M nitric acid to a 250 ml polytetrafluoroethylene bottle. The mixture would then be hand-shaken briefly and then sealed and heated to 60°C in a convection oven for 50 hours, then cooled to 25°C at 0.1°C per minute. The cap would then be removed from the bottle then the bottle was returned to the oven and heated from 60°C to 180°C at 0.1°C per minute, then held at 180°C for 12 hours, followed by cooling to 25°C at 2.5°C per minute. The dried gel would then be placed in a porcelain dish and heated in a furnace to 105°C at 0.9°C per minute, then to 160°C at 0.2°C per minute, then to 500°C at 0.5°C per minute then to 700°C at 0.1°C per minute. The furnace would then be held at 700°C for 1 hour then cooled back to 25°C at 10°C per minute. The heated material would be stored in a desiccator.

Celite 209 is a medium surface area 10-20 m²/g diatomaceous earth that is available from World Minerals Inc., headquartered in Santa Barbara, California, USA.

Celite 270 is a low surface area 4-6 m²/g diatomaceous earth, also available from World Minerals Inc.

Calcium polyphosphate glass can be prepared by heating 64 g of monobasic calcium phosphate monohydrate at 10°C per minute to 500°C and held at 500°C for 15 hours. The material would then be heated from 500°C to 1100°C at 10°C per minute then held at 1100°C for 1 hour. The molten polyphosphate glass would then be poured directly into 1 liter of deionized water. The resulting glass frit would then be dried at 110°C for 1 hour and milled in a corundum vibratory mill to a fine powder.

Siltex 18 - a 97% silica fiberglass cloth - SILTEX is a family of high performance textile fabric that is comprised of high purity, high strength amorphous silica fibers, woven into a strong, flexible fabric designed for use where severe temperature conditions exist.

Calcined Zr-Si glass that comprises alkali resistant (AR) glass fibers available from St. Gobain Group, Courbevoie France.

Hi-Sil 250 - a precipitated silica (silica gel) available from PPG Industries, Pittsburgh, Pennsylvania

Quartz sand that typically is 99% silica.

The hemostatic articles of the present invention offer a significant hemostatic effect while minimizing any issues involved with contact with the wound. Although the hemostatic articles have a significant loading of inorganic materials, the wound is no longer directly exposed to the adsorbents or the inorganic materials as when they are directly applied to the wound, thus allowing for the use of this product for treatment of minor though persistent bleeding as well as life-threatening wounds. In addition, the incorporation of the adsorbents or inorganic materials in a nonwoven sheet form can allow for the use of the product to effectively arrest bleeding during surgical procedures and thereby allow the surgeon to concentrate on the surgery rather than devote significant time to the control of bleeding.

In addition to non-woven sheets, other forms of materials are considered including films where a mixture of adsorbents or inorganic materials are combined with a polymer to form a film that may be applied to a wound for hemostatic purposes. A variety of adsorbents or inorganic materials may be used that provide a desired combination of hemostatic effect, lowered heat of adsorption and biocompatibility.

## Claims

1. A hemostatic article comprising a porous carrier and either an adsorbent or an inorganic material composition, wherein the article further comprises one or more hemostatic agents, one or more therapeutic agents or a combination thereof; **characterised in that** the porous carrier is a woven or non-woven fibrous article that is made from aramid fibers.

2. The hemostatic article of claim 1 wherein the article is in the form of a puff, a sponge, a ball, a suture, a powder, a gel, a foam, a film or a sheet.

3. The hemostatic article of claim 1 wherein said fibers are fibrillated.

4. The hemostatic article of claim 1 which comprises an adsorbent.

5. The hemostatic article of claim 1 which comprises an inorganic material.

6. The hemostatic article of claim 1 wherein said adsorbent is a zeolite molecular sieve or a non-zeolite molecular sieve and said inorganic materials are selected from the group consisting of diatomaceous earth, glass powder or fibers, precipitated or fumed silica, kaolin and montmorillonite clays, and Ca exchanged permutites.

7. The hemostatic article of claim 4 wherein the adsorbent is selected from the group consisting of zeolite X, Y, A, beta and mixtures thereof.

8. The hemostatic article of claim 1 further comprising calcium ions or bioactive fillers to release calcium, silver ions or a mixture thereof.

9. The hemostatic article of claim 4 wherein the absorbent is a naturally occurring zeolite molecular sieve, a synthetic zeolite molecular sieve or a non-zeolite molecular sieve.

10. The hemostatic article of claim 9 wherein the adsorbent is a non-zeolite molecular sieve.

11. The hemostatic article of claim 1 wherein said hemostatic agent or therapeutic agent is selected from the group consisting of analgesics, steroids, antihistamines, anesthetics, bactericides, disinfectants, fungicides, vasoconstrictors, hemostatics, chemotherapeutic drugs, antibiotics, keratolytics, cauterizing agents, antiviral drugs, epidermal growth factor, fibroblast growth factors, transforming growth factors, glycoproteins, fibrinogen, fibrin, humectants, preservatives, lymphokines; cytokines, odor controlling materials, vitamins, and clotting factors.

12. The hemostatic article of claim 1 wherein said article comprises a porous carrier that comprises paper.

13. The hemostatic article as claimed in claim 4 wherein the absorbent is a calcium zeolite.

14. The hemostatic article of claim 12 wherein the paper is prepared from aramid pulp and the inorganic material or molecular sieve filler content is from 75 to 90 wt%.

15. The hemostatic article of claim 1 wherein the article is in the form of a fabric loaded with said adsorbent or inorganic material composition, and wherein said fabric contains from about 50 wt.% to about 80 wt.% of the adsorbent or the inorganic material composition.

## Patentansprüche

1. Hämostatischer Gegenstand, umfassend einen porösen Träger und entweder ein Adsorptionsmittel oder eine anorganische Materialzusammensetzung, wobei der Gegenstand ferner ein oder mehrere hämostatische Mittel, ein oder mehrere therapeutische Mittel oder eine Kombination davon umfasst; **dadurch gekennzeichnet, dass** der poröse Träger ein gewebter oder nichtgewebter Fasergegenstand ist, der aus Aramidfasern besteht.

2. Hämostatischer Gegenstand gemäß Anspruch 1, wobei der Gegenstand in der Form eines Bauschs, eines Schwamms, einer Kugel, eines Nahtfadens, eines Pulvers, eines Gels, eines Schaumstoffs, eines Films oder eines Blatts vorliegt.

3. Hämostatischer Gegenstand gemäß Anspruch 1, wobei die Fasern fibrilliert sind.

4. Hämostatischer Gegenstand gemäß Anspruch 1, umfassend ein Adsorptionsmittel.

5. Hämostatischer Gegenstand gemäß Anspruch 1, umfassend ein anorganisches Material.

6. Hämostatischer Gegenstand gemäß Anspruch 1, wobei das Adsorptionsmittel ein Zeolith-Molekularsieb oder ein Nichtzeolith-Molekularsieb ist und die anorganischen Materialien ausgewählt sind aus der Gruppe bestehend aus Diatomeenerde, Glaspulver oder -fasern, präzipitiertem oder pyrogenem Siliciumdioxid, Kaolin- und Montmorillonit-Tonen und Ca-ausgetauschten Permutiten.

7. Hämostatischer Gegenstand gemäß Anspruch 4, wobei das Adsorptionsmittel ausgewählt ist aus der Gruppe bestehend aus Zeolith X, Y, A, beta und Gemischen davon.

8. Hämostatischer Gegenstand gemäß Anspruch 1, ferner umfassend Calciumionen oder bioaktive Füllstoffe zum Freisetzen von Calcium, Silberionen oder einem Gemisch davon.

9. Hämostatischer Gegenstand gemäß Anspruch 4, wobei das Absorptionsmittel ein natürlich vorkommendes Zeolith-Molekularsieb, ein synthetisches Zeolith-Molekularsieb oder ein Nichtzeolith-Molekularsieb ist.

10. Hämostatischer Gegenstand gemäß Anspruch 9, wobei das Adsorptionsmittel ein Nichtzeolith-Molekularsieb ist.

11. Hämostatischer Gegenstand gemäß Anspruch 1, wobei das hämostatische Mittel oder therapeutische Mittel ausgewählt ist aus der Gruppe bestehend aus Analgetika, Steroiden, Antihistaminika, Anästhetika, Bakteriziden, Desinfektionsmitteln, Fungiziden, Vasokonstriktoren, Hämostatika, chemotherapeutischen Arzneimitteln, Antibiotika, Keratolytika, kauterisierenden Mitteln, antiviralen Arzneimitteln, epidermalem Wachstumsfaktor, Fibroblasten-Wachstumsfaktoren, transformierenden Wachstumsfaktoren, Glycoproteinen, Fibrinogen, Fibrin, Feuchthaltemitteln, Konservierungsmitteln, Lymphokinen, Zytokinen, geruchskontrollierenden Materialien, Vitaminen und Gerinnungsfaktoren.

12. Hämostatischer Gegenstand gemäß Anspruch 1, wobei der Gegenstand einen porösen Träger umfasst, der Papier umfasst.

13. Hämostatischer Gegenstand gemäß Anspruch 4, wobei das Absorptionsmittel ein Calciumzeolith ist.

14. Hämostatischer Gegenstand gemäß Anspruch 12, wobei das Papier aus Aramidpulpe hergestellt ist und der Gehalt an anorganischem Material oder Molekularsieb-Füllstoff von 75 bis 90 Gew.-% beträgt.

15. Hämostatischer Gegenstand gemäß Anspruch 1, wobei der Gegenstand in der Form eines mit dem Adsorptionsmittel oder der anorganischen Materialzusammensetzung beladenen Gewebes vorliegt und wobei das Gewebe von etwa 50 Gew.-% bis etwa 80 Gew.-% an dem Adsorptionsmittel oder der anorganischen Materialzusammensetzung enthält.

## Revendications

1. Article hémostatique comprenant un support poreux et une composition soit d'adsorbant, soit de matériau inorganique, l'article comprenant en outre un ou plusieurs agents hémostatiques, un ou plusieurs agents thérapeutiques, ou une combinaison de ceux-ci ; **caractérisé en ce que** le support poreux est un article fibreux tissé ou non tissé qui se compose de fibres d'aramide.

2. Article hémostatique selon la revendication 1, l'article étant sous la forme d'un tampon, d'une éponge, d'une boule, d'une suture, d'une poudre, d'un gel, d'une mousse, d'un film ou d'une feuille.

3. Article hémostatique selon la revendication 1, dans lequel lesdites fibres sont fibrillées.

4. Article hémostatique selon la revendication 1, lequel comprend un adsorbant.

5. Article hémostatique selon la revendication 1, lequel comprend un matériau inorganique.

6. Article hémostatique selon la revendication 1, dans lequel ledit adsorbant est un tamis moléculaire de zéolite ou un tamis moléculaire qui n'est pas une zéolite et lesdits matériaux inorganiques sont sélectionnés dans le groupe constitué de la terre de diatomées, de la poudre ou des fibres de verre, de la silice précipitée ou pyrogénée, d'argiles de type kaolin et montmorillonite, et de permutites ayant fait l'objet d'un échange d'ions calcium.

7. Article hémostatique selon la revendication 4, dans lequel l'adsorbant est sélectionné dans le groupe constitué de zéolites X, Y, A, bêta, et de mélanges de celles-ci.

8. Article hémostatique selon la revendication 1, comprenant en outre des ions calcium ou des charges bioactives pour libérer du calcium, des ions argent, ou un mélange de ceux-ci.

9. Article hémostatique selon la revendication 4, dans lequel l'absorbant est un tamis moléculaire de zéolite naturelle, un tamis moléculaire de zéolite synthétique, ou un tamis moléculaire qui n'est pas une zéolite.

10. Article hémostatique selon la revendication 9, dans lequel l'adsorbant est un tamis moléculaire qui n'est pas une zéolite.

11. Article hémostatique selon la revendication 1, dans lequel ledit agent hémostatique ou agent thérapeutique est sélectionné dans le groupe constitué d'analgésiques, de stéroïdes, d'antihistaminiques, d'anesthésiques, de bactéricides, de désinfectants, de fongicides, de vasoconstricteurs, d'hémostatiques, de médicaments de chimiothérapie, d'antibiotiques, de kératolytiques, d'agents de cautérisation, de médicaments antiviraux, d'un facteur de croissance épidermique, de facteurs de croissance des fibroblastes, de facteurs de croissance transformants, de glycoprotéines, de fibrinogène, de fibrine, d'humectants, de conservateurs, de lymphokines, de cytokines, de matériaux anti-odeurs, de vitamines, et de facteurs de coagulation.

12. Article hémostatique selon la revendication 1, ledit article comprenant un support poreux qui comprend du papier.

13. Article hémostatique selon la revendication 4, dans lequel l'absorbant est une zéolite calcique.

14. Article hémostatique selon la revendication 12, dans lequel le papier est préparé à partir d'une pâte d'aramide et la teneur en charge sous forme de matériau inorganique ou de tamis moléculaire est de 75 à 90 % en poids.

15. Article hémostatique selon la revendication 1, l'article étant sous la forme d'un tissu chargé de ladite composition d'adsorbant ou de matériau inorganique, et ledit tissu contenant d'environ 50 % en poids à environ 80 % en poids de la composition d'adsorbant ou de matériau inorganique.
